# EUROPEAN PATENT APPLICATION

(11) **EP 3 733 218 A1**
(43) Date of publication of application: **04.11.2020**
(21) Application number: 20182793.8
(22) Date of filing: 18.04.2017
(51) Int. Cl.: A61L 15/32, A61L 15/22, A61L 15/42, A61L 15/58, B32B 9/02, B32B 9/04, B32B 5/32, B32B 5/24, B32B 5/18, B32B 5/02

(54) **SHEET-LIKE HEMOSTATIC MATERIAL EMPLOYING POLY-Y-GLUTAMIC ACID, AND METHOD OF MANUFACTURING SAME**

(30) Priority: 20.04.2016 JP 2016084551; 20.04.2016 JP 2016084552; 11.04.2017 JP 2017078019
(62) Divisional of application: 17785978.2
(71) Applicant: Nipro Corporation, Osaka-shi, Osaka 531-8510 (JP)
(72) Inventor: NAKAMURA, Yusuke, Osaka, Osaka 531-8510 (JP); SUGAHARA, Yoshikatsu, Osaka, Osaka 531-8510 (JP); MATSUDA, Kazuhisa, Osaka, Osaka 531-8510 (JP)
(74) Representative: Dantz, Jan Henning

(57) **Abstract**

A sheet-like hemostatic material (10A to 10E) is provided with: an adhesive layer (11) which serves as an affixing surface to a bleeding site and which is formed from at least poly-γ-glutamic acid (y-PGA); and a covering layer (12) which is a sheet formed from at least one biodegradable material (covering material) other than γ-PGA, and which serves as an opposite surface to the affixing surface. At least one of the adhesive layer (11) and the covering layer (12) is a single sheet. If the adhesive layer (11) is a single sheet, the adhesive layer (11) should be a sponge-like sheet, and if the adhesive layer (11) is a partial layer, the adhesive layer (11) should be a sponge-like sheet or a nonporous layer. Alternatively, in another sheet-like hemostatic material, a sheet which is formed from at least poly-γ-glutamic acid (γ-PGA) and to which at least one of a saccharide and collagen has been added is provided as the adhesive layer.

## Description

### Technical Field

The present invention relates to a sheet-like hemostatic material employing poly-γ-glutamic acid, which can be suitably used for local hemostasis, and a method of manufacturing the sheet-like hemostatic material.

### Background Art

For example, when local bleeding occurs in a surgical operation or the like, use of a sheet-like hemostatic material is known as one of methods for hemostasis treatment. The sheet-like hemostatic material is formed by molding into a sheet shape a medical material which is decomposable in vivo. Typically, the material used for the sheet-like hemostatic material is a material derived from animal proteins (animal material), a material derived from polysaccharides (polysaccharide material), a material derived from synthetic polymers (polymer material), and the like. It is known that typical examples of the animal material include fibrin, collagen, and the like; typical examples of the polysaccharide material include a cellulose type material, a starch type material, and the like; and examples of the polymer material include a polyacrylic acid type polymer material and the like.

As commercially available representative sheet-like hemostatic materials, there are known "TachoSil" (registered trademark, CSL Behring K.K.) containing an animal material and "SURGICEL" (registered trademark, Johnson & Johnson K.K.) containing a polysaccharide material. The TachoSil is a slightly hard sponge-like sheet containing human-derived fibrinogen and thrombin and has high adhesive strength and excellent hemostatic performance. The SURGICEL is made of regenerated oxidized cellulose derived from wood pulp, and includes a gauze type, a cotton type, and a knit type (NU-KNIT). Particularly, the knit type is suitably used as a sheet-like hemostatic material.

Furthermore, as disclosed in JP-A-2014-004066 or JP-A-2014-005219, it has been recently proposed to improve the flexibility of a sheet-like hemostatic material. The sheet-like hemostatic materials described in these Patent Literatures are formed from a sheet in which fibrinogen is immobilized on a fiber molded body made of biodegradable polymer fibers such as poly lactic acid and a sheet in which thrombin is immobilized on a fiber molded body similarly made of biodegradable polymer fibers. Improvement of hemostasis is aimed at supporting fibrinogen and thrombin on separate fiber molded bodies. Further, by changing the density of the bulk density of the fiber molded body (JP-A-2014-004066), or by forming a fiber molded body by combining fiber structure layers having different bulk densities (JP-A-2014-005219), the flexibility of the fiber molded body as the base material is improved.

### Citation List

### Patent Literature

CN 105 056 280 A discloses an absorbable hemostatic material and a method for preparing hemostatic material which involves taking calcium polyglutamic acid, dispersing with water and auxiliary material, freeze drying and sterilizing to obtain fa finished product.

### Summary of Invention

### Technical Problem

Incidentally, use of a sheet-like hemostatic material for hemostatic treatment in laparoscopic surgery has been recently studied. However, the conventional sheet-like hemostatic material is not sufficient in flexibility or handleability (or in both) for use in laparoscopic surgery.

In laparoscopic surgery, first, an instrument having a tubular part called a trocar is inserted in advance into the abdomen, and then a laparoscope and a surgical instrument are inserted into the trocar. Since the surgical field (surgical site) photographed with the laparoscope is magnified and displayed on the monitor, the surgeon performs surgery using surgical instruments while observing the monitor. Since the inner diameter of the trocar is usually about 5 to 12 mm, the sheet-like hemostatic material is required to have flexibility enough to be capable of being folded or rolled so that the hemostatic sheet can be inserted into such a small hole. Also, surgical instruments used for laparoscopic surgery will be operated in a small space within the abdominal cavity via the trocar. Therefore, such a sheet-like hemostatic material is also required to have good handleability.

Although the TachoSil has good hemostatic performance, it has a slightly hard sponge shape as described above, so it has no flexibility to be inserted into the trocar. Like the TachoSil, the sheet-like hemostatic materials disclosed in Patent Literatures 1 and 2 all contain fibrinogen. Fibrinogen becomes fibrin by the action of thrombin, and this fibrin reacts biochemically to realize hemostasis. Therefore, the sheet-like hemostatic material containing fibrinogen is strongly fixed if once affixed inside the abdominal cavity, and it becomes difficult to peel off and reapply the hemostatic material. In laparoscopic surgery, it is necessary to perform surgery by making full use of surgical instruments via the trocar, so unlike usual surgery performed by the operator's own hand, there are many cases where the hemostatic material cannot be affixed to an appropriate position. Therefore, it is difficult to say that a fibrinogen-based sheet-like hemostatic material has sufficient handleability when used for laparoscopic surgery.

Here, the SURGICEL NU-KNIT is an oxidized cellulose type sheet-like hemostatic material, which is more flexible than TachoSil and does not use fibrinogen, so it is not affixed firmly. However, the SURGICEL achieves hemostasis by contacting the blood to form a gelatinous clot. Therefore, after affixing to the hemostasis site, it is necessary to hold the SURGICEL for several minutes until a clot is formed. Thus, it is difficult to say that the SURGICEL NU-KNIT also has sufficient handleability for use in laparoscopic surgery.

In addition, since the fibrinogen-based sheet-like hemostatic material is a preparation (blood preparation) containing a component derived from human, risk of infection etc. occurs. Therefore, upon use, it is necessary to exchange with the user (patient) risk explanation and consent form indicating that the fibrinogen-based sheet-like hemostatic material is a human-derived preparation (blood preparation). Furthermore, since fibrinogen is a reactive protein and thrombin is an enzyme, in the case of TachoSil, low temperature preservation at 10°C or less is required. Although the main component of the SURGICEL is an oxidized cellulose, preservation at 25°C or less is required. Therefore, it is difficult to say that conventional sheet-like hemostatic materials on the market have sufficient handleability from the viewpoint that they are all restricted in transportation and preservation.

The present invention has been made to solve such problems, and it is an object of the present invention to provide a sheet-like hemostatic material that realizes good hemostatic performance without the need of containing fibrinogen and further realizes good flexibility and handleability.

### Solution to Problem

In order to solve the problems, the sheet-like hemostatic material according to the present invention is provided having the features of claim 1 and a method comprising the features of claim 10.

According to the above configuration to make the affixing surface of the sheet-like hemostatic material to be an adhesive layer formed from poly-γ-glutamic acid (γ-PGA), good adhesiveness to biological tissue and good hemostatic performance can be realized with γ-PGA of the adhesive layer without containing animal materials such as fibrinogen. Further, since γ-PGA has good adhesiveness without accompanying biochemical reaction, even in the case where a hemostatic material cannot be affixed to, for example, an appropriate position, it becomes possible to reapply to a biological tissue unlike the conventional sheet-like hemostatic material.

In the sheet-like hemostatic material having the above configuration, a covering layer which is provided on an opposite surface to the affixing surface and is formed from at least one biodegradable material other than the poly-γ-glutamic acid may be further provided and at least one of the adhesive layer and the covering layer may be a single sheet.

According to the above configuration, an adhesive layer formed from poly-γ-glutamic acid (γ-PGA) is provided on the affixing surface, and on the opposite surface which is a non-affixing surface, a covering layer formed from a biodegradable material (covering material) other than the γ-PGA is provided. Therefore, by containing the γ-PGA of the adhesive layer, good adhesiveness to biological tissues and good hemostatic performance can be realized without containing animal materials such as fibrinogen, and by containing the covering material of the covering layer, it is possible to effectively suppress unintentional adhesiveness of the sheet-like hemostatic material to the tissues other than the affixing site or to the surgical instruments.

In addition, according to the above configuration, since at least one of the adhesive layer and the covering layer is a single sheet, the adhesive layer or the covering layer, or both, can function as the base material of the sheet-like hemostatic material. Furthermore, since the adhesive layer is made of γ-PGA, by appropriately selecting the covering material to be the covering layer, the sheet-like hemostatic material of the present invention can be stored for a longer time at room temperature as compared with the conventional sheet-like hemostatic material.

Further, in the sheet-like hemostatic material having the above configuration, the adhesive layer may be a sponge-like sheet when the adhesive layer is the single sheet. Alternatively, in the sheet-like hemostatic material having the above configuration, when the adhesive layer is a partial layer which is not the single sheet, the adhesive layer may be a sponge-like sheet formed from at least poly-γ-glutamic acid or a nonporous layer.

According to the above configuration, if the adhesive layer is sponge-like or the adhesive layer is a non-porous partial layer, the adhesive layer can achieve appropriate flexibility or good flexibility while the covering layer can also achieve flexibility, so that it becomes possible to realize good flexibility as the sheet-like hemostatic material. Therefore, breakage of the hemostatic material can be effectively suppressed even if the sheet-like hemostatic material is rounded or bent. Further, according to the above configuration, since the hemostatic material has characteristics of good flexibility, reapplication property, and storage at room temperature, good handleability can be achieved.

In the sheet-like hemostatic material having the above configuration, the covering layer may be a sponge-like sheet formed from at least a crosslinked collagen.

In the sheet-like hemostatic material having the above configuration, both the adhesive layer and the covering layer may be each a single sheet, or the covering layer may be a partial layer provided partially on the opposite surface.

In the sheet-like hemostatic material having the above configuration, at least a part of the adhesive layer and the covering layer may have different colors from each other.

In the sheet-like hemostatic material having the above configuration, a weight ratio of the adhesive layer may be in a range of 10 to 90% by weight based on a total weight of the sheet-like hemostatic material.

Further, in the sheet-like hemostatic material having the above configuration, at least one intermediate layer interposed between the adhesive layer and the covering layer may be provided, and these respective layers may be integrally fixed.

In addition, in order to solve the problems, a method of manufacturing a sheet-like hemostatic material according to the present invention includes a step of laminating a first layered body formed from at least poly-γ-glutamic acid and a second layered body formed from at least one biodegradable material other than the poly-γ-glutamic acid to form a laminate, and a step of adhering or pressure-bonding the laminate to form a sheet-like hemostatic material including an adhesive layer formed from at least the poly-γ-glutamic acid and a covering layer formed from the biodegradable material.

In the method of manufacturing a sheet-like hemostatic material having the above configuration, the first layered body may be a first sponge-like porous body formed form at least poly-γ-glutamic acid, a first sponge-like sheet obtained by applying pressure to the first sponge-like porous body, or a first partial sheet which is formed from at least poly-γ-glutamic acid and is not a single sheet, and the first partial sheet is sponge-like or non-sponge-like nonporous, and the second layered body may be a second sponge-like porous body formed from the biodegradable material, a second sponge-like sheet obtained by applying pressure to the second sponge-like porous body, or a second partial sheet which is formed from the biodegradable material and is not a single sheet.

In the method of manufacturing a sheet-like hemostatic material having the above configuration, the laminate may be formed by first applying pressure to the second sponge-like porous body to form the second sponge-like sheet, laminating and pressure-bonding the second sheet on the first sponge-like porous body.

In addition, in another sheet-like hemostatic material according to the present invention, a sheet which is formed from at least poly-γ-glutamic acid and to which a saccharide has been added is provided as the adhesive layer in order to solve the problems.

According to the above configuration, the adhesive layer is formed from a sheet containing poly-γ-glutamic acid (γ-PGA) as a main component to which a saccharide is added. A γ-PGA sheet consisting essentially of γ-PGA alone does not have flexibility and breaks when it is intended to be deformed, but a γ-PGA sheet containing a saccharide can exhibit good flexibility. Therefore, containing γ-PGA in the adhesive layer can realize good adhesiveness to biological tissue and good hemostatic performance without containing animal materials such as fibrinogen, and breakage of the adhesive layer can be effectively suppressed even if the sheet-like hemostatic material is rounded or bent.

Furthermore, since γ-PGA has good adhesiveness without accompanying biochemical reactions, it becomes possible to reapply to the biological tissue as compared to the conventional sheet-like hemostatic material. In addition, since the adhesive layer is formed from γ-PGA, the sheet-like hemostatic materials of the present invention can be stored for a longer time at room temperature as compared with the conventional sheet-like hemostatic material. That is, according to the above configuration, since the sheet-like hemostatic material of the present invention has characteristics of good flexibility, reapplication, and storage at room temperature, good handleability can be realized.

In the sheet-like hemostatic material having the above configuration, the saccharide may be at least one of a monosaccharide, an oligosaccharide, and a polysaccharide.

In the sheet-like hemostatic material having the above configuration, the saccharide may be added in an amount ranging from 10 to 90% by weight based on a total weight of the sheet.

Further, in the sheet-like hemostatic material having the above configuration, collagen may have been added to the sheet.

Furthermore, the present invention also includes a sheet-like hemostatic material provided with a sheet as an adhesive layer which is formed from at least poly-γ-glutamic acid and to which collagen has been added.

In addition, in order to solve the problems, a method of manufacturing a sheet-like hemostatic material according to the present invention includes a step of forming an adhesive layer by adding at least one of a saccharide and collagen to poly-γ-glutamic acid to form into a sheet.

These and other objects, features, and advantages of the present invention will become apparent from the following detailed description of the preferred embodiments with reference to the accompanying drawings.

### Advantageous Effects of Invention

With the configuration, the present invention exhibits effects such that it is possible to realize good hemostatic performance without containing fibrinogen in the sheet-like hemostatic material, and furthermore, good flexibility and handleability.

### Brief Description of Drawings

Fig. 1A and Fig. 1B are schematic cross-sectional views showing schematic structures of the sheet-like hemostatic materials according to Embodiment 1 of the present disclosure.
Figs. 2A, 2B, and 2C are schematic cross-sectional views showing modified examples of the sheet-like hemostatic materials according to Embodiment 1.
Figs. 3A, 3B, and 3C are schematic plan views showing respectively an example of a surface opposite to the affixing surface in the sheet-like hemostatic material shown in Fig. 2A.

### Description of Embodiments

The sheet-like hemostatic material according to the present disclosure is configured by containing at least poly-γ-glutamic acid. Specifically, such hemostatic materials include a sheet-like hemostatic material provided with an adhesive layer which is formed from at least poly-γ-glutamic acid and is provided on a surface to be affixed to a bleeding site, and a sheet-like hemostatic material including a sheet as an adhesive layer which is formed from at least poly-γ-glutamic acid and to which a saccharide has been further added. The sheet-like hemostatic material of each of these configurations will be specifically described.

### (Embodiment 1)

The sheet-like hemostatic material according to the present disclosure may take a configuration in which an adhesive layer is provided on a surface to be affixed to a bleeding site and is formed from at least poly-γ-glutamic acid, but preferably may take a configuration in which in addition to the adhesive layer, a covering layer which is provided on the opposite surface of the affixing surface to the bleeding site and is formed from at least one kind of biodegradable materials other than the poly-γ-glutamic acid and in which at least one of the adhesive layer and the covering layer is a single sheet. Hereinafter, representative embodiments of the present disclosure will be specifically described.

### [Sheet-Like Hemostatic Material]

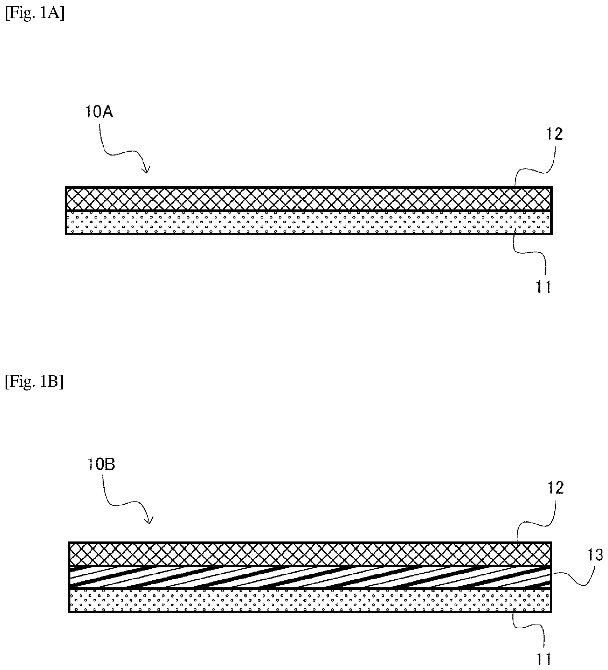

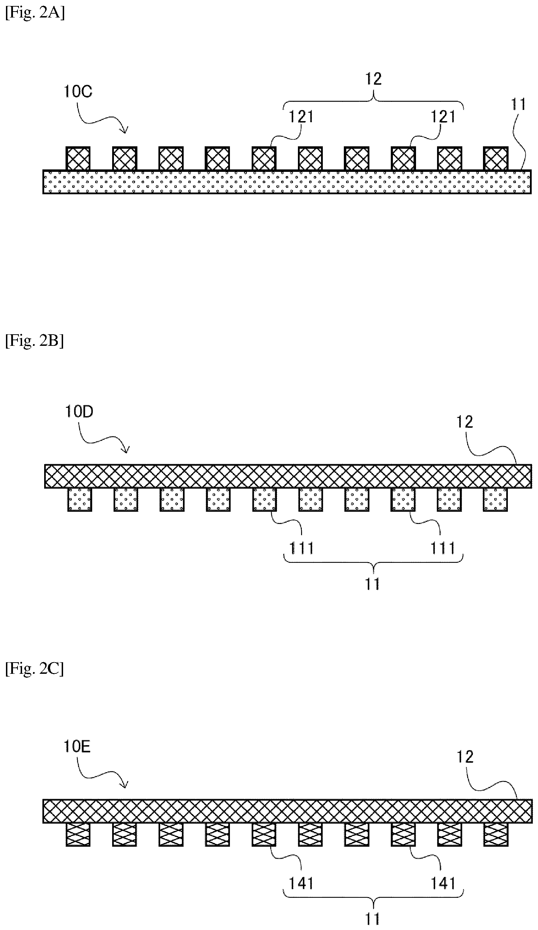

Fig. 1A or Fig. 1B schematically shows a cross-sectional structure of a sheet-like hemostatic material 10A or 10B as a representative example of a sheet-like hemostatic material according to the present disclosure. Figs. 2A to 2C schematically show cross-sectional structures of sheet-like hemostatic materials 10C to 10E as another example of the sheet-like hemostatic material according to the present disclosure. These sheet-like hemostatic materials 10A to 10E are provided with an adhesive layer 11 and a covering layer 12. As described above, the adhesive layer 11 may be a sheet containing poly-γ-glutamic acid (y-PGA) as a main component. In this embodiment, as shown in Fig. 1A, Fig. 1B, Fig. 2A or Fig. 2B, the adhesive layer 11 is a sponge-like (porous) sheet or, as shown in Fig. 2C, the adhesive layer 11 may be a partial layer to be described later when it is not sponge-like and nonporous. The covering layer 12 may be a sheet containing a biodegradable material other than γ-PGA as a main component, and in this embodiment, the covering layer 12 is a sponge-like sheet containing a crosslinked collagen as a main component as described later. In the following description, the biodegradable material other than γ-PGA is referred to as "covering material" for convenience.

The adhesive layer 11 forms an affixing surface (back side) for affixing the sheet-like hemostatic materials 10A to 10E to a bleeding site of the biological tissue or the like and has good adhesiveness to the biological tissue and the like. As shown in Fig. 1A, Fig. 1B or Fig. 2A, the adhesive layer 11 may be provided as a single layer covering the entire surface of the affixing surface, or as shown in Fig. 2B or Fig. 2C, the adhesive layer 11 may be provided as a partial layer that partially forms the affixing surface. Similarly, the covering layer 12 is provided on at least a part of the opposite surface (front side) opposite to the affixing surface when the sheet-like hemostatic material 10A is affixed to the bleeding site. The covering layer 12 may be provided as a single layer covering the entire surface of the opposite surface as shown in Fig. 1A or 1B, or as a partial layer partially covering the opposite surface as shown in Fig. 2A.

Even though the covering layer 12 may be either a single layer or have a partially covered configuration, unlike the adhesive layer 11, the covering layer 12 does not have adhesiveness to biological tissues or the like or has sufficiently smaller adhesiveness than the adhesive layer 11. In other words, the covering layer 12 may have a configuration that allows the opposite surface of the adhesive layer 11 to be a "non-adhesive surface". Thereby, when the sheet-like hemostatic materials 10A to 10E are to be affixed to the bleeding site, it is possible to effectively suppress the possibility of inadvertent affixing to the site other than the bleeding site or the surgical instrument or the like. That is, the covering layer 12 has a function of covering the other surface so as to suppress the adhesiveness thereof so that only one surface of the sheet-like hemostatic materials 10A to 10E becomes the affixing surface.

As described above, the adhesive layer 11 is a sponge-like sheet formed from γ-PGA in the case of the sheet-like hemostatic materials 10A to 10D shown in Figs. 1A, 1B, 2A or 2B, but the covering layer 12 may be any sheet as long as it is a sheet formed from a covering material and at least it can exhibit flexibility when forming the sheet-like hemostatic materials 10A to 10E. However, the covering layer 12 is also preferably a sponge-like sheet formed from a covering material. If the covering layer 12 is a sponge-like sheet, it is possible to impart good flexibility to the covering layer 12 regardless of the specific type of the covering materials.

The sponge-like sheet as used herein may be a flexible porous sheet, and its specific configuration is not particularly limited. As a specific configuration of the sponge-like sheet, it can include a porous material (porous body) in which a section having a large number of gaps (pores or vacuoles) of uniform or nonuniform size is continuously or discontinuously dispersed when visually observed or observed under a microscope. It is to be noted that the non-sponge-like non-porous layer or sheet means a sheet or a layer integrally formed from γ-PGA or a composition containing γ-PGA as a main component, including the state where the section having a large number of the gaps (pores or vacuoles etc.) is substantially absent (the existence of the section can be substantially ignored even if the section exists slightly).

The method of processing at least γ-PGA or γ-PGA and the covering material into a sponge-like sheet is not particularly limited, and a known method can be suitably used. Specifically, for example, a solution of γ-PGA or a covering material (or a composition thereof) is poured into a mold having a predetermined shape and dried by a drying method such as natural drying, vacuum drying, and vacuum freeze drying to obtain a sponge-like porous body, which is then applied by a pressure to form a sponge-like sheet. Among them, from the viewpoint of forming the porous material more uniformly, a vacuum freeze-drying method can be preferably mentioned, but the drying method is not limited thereto.

As a vacuum freeze-drying method, from the viewpoint of ease of production, it includes, for example, a method in which a solution of about 0.05 to 30% by weight of γ-PGA or the covering material is preliminarily frozen and then dried under a pressure of about 10.6 Pa (about 0.08 Torr) or less but is not limited thereto. After freeze-drying, a sponge-like porous body as a molded article can be obtained by removal from the mold. The method of molding the sponge-like porous body into a sheet shape is also not particularly limited, and for example, a method of compressing with a press or the like can be mentioned.

The sheet-like hemostatic material 10A shown in Fig. 1A has a two-layer structure configured only by the adhesive layer 11 and the covering layer 12, but the present disclosure is not limited thereto, and the sheet-like hemostatic material of the present disclosure may have a multilayer structure of three or more layers like the sheet-like hemostatic material 10B as shown in Fig 1B. That is, in the sheet-like hemostatic material according to the present disclosure, the laminated structure is not particularly limited as long as one surface (back surface) is an affixing surface formed from γ-PGA and the other surface (front surface) is a non-affixing surface (front surface) covered with a covering material (biodegradable material other than γ-PGA).

Since the sheet-like hemostatic material 10B shown in Fig. 1B has an intermediate layer 13 between the adhesive layer 11 and the covering layer 12, it has a three-layer structure. The intermediate layer 13 may be a layer interposed between the adhesive layer 11 and the covering layer 12 and may be one layer like the sheet-like hemostatic material 10B or two or more layers. The concrete structure of the intermediate layer 13 is not particularly limited, and any intermediate layer may be used as long as it can impart various functions and the like required for use in the sheet-like hemostatic material 10B.

For example, it is assumed that the sheet-like hemostatic materials 10A to 10E are sewn to the vicinity of the bleeding site. In the case where the covering layer 12 is a sponge-like sheet formed from a crosslinked collagen, since the adhesive layer 11 is a sponge-like sheet formed from γ-PGA, it is not easy to sew the sheet-like hemostatic material 10A having no intermediate layer 13 to a biological tissue or the like. Therefore, in the sheet-like hemostatic material 10B, if a mesh made of a thread material of a biodegradable material is adopted as the intermediate layer 13, sewing to the biological tissue or the like becomes easy. Examples of the other intermediate layer 13 can include a fixing layer for improving the adhesiveness state between the adhesive layer 11 and the covering layer 12, or a reinforcing layer for improving the strength of the sheet-like hemostatic material 10B.

Furthermore, in the present disclosure, like the sheet-like hemostatic material 10A shown in Fig. 1A, both the adhesive layer 11 and the covering layer 12 are configured as a single sheet, but the present disclosure is not limited thereto. In the present disclosure, at least one of the adhesive layer 11 and the covering layer 12 may be a single sheet (both may be a single sheet), and the other may not be a single sheet.

As used herein, the term "single sheet" means an integral single planar member having a two-dimensional spread and does not mean a member aggregate that is collectively configured by a plurality of planar members like a partial layer described later. As the layers forming the sheet-like hemostatic materials 10A to 10E, the adhesive layer 11 or the covering layer 12, which is formed as a "single sheet", means a layer of a planar member that can substantially cover the entire surface of the affixing surface or the opposite side thereof so that the bleeding from the bleeding site can be appropriately stopped when the sheet-like hemostatic materials 10A to 10E are affixed to the bleeding site.

A configuration in which one of the adhesive layer 11 and the covering layer 12 is not a single sheet, that is, a configuration in which one of the adhesive layer 11 and the covering layer 12 is a partial layer will be specifically described. For example, like the sheet-like hemostatic material 10C shown in Fig. 2A, the covering layer 12 may be provided on the opposite surface as a plurality of partial covering layers (partial layers) 121 partially covering the opposite surface. Alternatively, like the sheet-like hemostatic material 10D shown in Fig. 2B, the adhesive layer 11 may be provided on the adhesive surface as a plurality of partial adhesive layers (partial layers) 111 partially covering the adhesive surface. The partial adhesive layer 111 is a sponge-like sheet. Alternatively, like the sheet-like hemostatic material 10E shown in Fig. 2C, the adhesive layer 11 may be provided on the adhesive surface as a plurality of partial adhesive layers 141 which are nonporous layers. The sheet-like hemostatic materials 10A to 10E require a single sheet (base material layer) as a base material in order to maintain the sheet shape. In the sheet-like hemostatic material 10A or 10B, all of the layers are formed from a single sheet, but since the one layer of the sheet-like hemostatic material 10C, 10D or 10E is a partial layer, the other layer is a base material layer, so it is necessary to form the hemostatic material as a single sheet.

In addition, since the adhesive layer 11 is formed in a sponge shape, flexibility can be exhibited as described later. Therefore, even if the adhesive layer 11 is a single sheet as shown in Fig. 1A or Fig. 1B, or even if the adhesive layer 11 is configured by the partial adhesive layer 111 as shown in Fig. 2A, good flexibility can be exhibited. However, the present disclosure is not limited to thereto. For example, as shown in Fig. 2C, the adhesive layer 11 may not be in the form of a sponge as long as the adhesive layer 11 is configured by a partial layer.

A simple sheet formed from γ-PGA as a main component (i.e., non-porous sheet which is not sponge-like) lacks flexibility as exemplified in Comparative Example 3 described later. However, if the adhesive layer 11 is formed as a partial adhesive layer 141 even if it is nonporous and contains γ-PGA as a main component, the partial adhesive layer 141 can exhibit flexibility since the partial adhesive layer 141 is supported by the covering layer 12. Therefore, the sheet-like hemostatic material 10E becomes to have sufficient flexibility.

In other words, the present disclosure includes (1) a sheet-like hemostatic material 10A or 10B which is a sponge-like sheet (single sheet) formed from at least γ-PGA, including an adhesive layer 11 which forms a surface for affixing to a bleeding site and a covering layer 12 which is a sheet (single sheet) formed from at least one biodegradable material other than γ-PGA and configures a surface opposite to the affixing surface, (2) a sheet-like hemostatic material 10C including an adhesive layer 11, which is a sponge-like sheet provided as a single layer (single sheet) to cover the entire adhesive surface (to form an adhesive surface), and a covering layer 12 configured as a partial covering layer 121 which is partially provided on the opposite surface, (3) a sheet-like hemostatic material 10D, in which a covering layer 12 is provided as a single layer (a single sheet) covering the entire surface of the opposite surface of an adhesive layer 11 (to form an opposite surface) and the adhesive layer 11 is partially provided on the affixing surface to form a sponge-like partial adhesive layer 111, and (4) a sheet-like hemostatic material 10E, in which a covering layer 12 is provided as a single layer (a single sheet) covering the entire surface of the opposite surface of an adhesive layer 11 (to form an opposite surface) and the adhesive layer 11 is partially provided on the affixing surface to form a nonporous partial adhesive layer 141.

In the present disclosure, if the adhesive layer 11 is a sponge-like sheet formed from γ-PGA, good flexibility can be exhibited like the sheet-like hemostatic material 10A or 10B even if the adhesive layer 11 is a single sheet. Furthermore, by forming one of the adhesive layer 11 and the covering layer 12 as a base material layer formed from a single sheet and the other as a partial layer like the sheet-like hemostatic material 10C or 10D, it is possible to further improve the flexibility of the sheet-like hemostatic material. Even if the adhesive layer 11 is a non-sponge-like nonporous shape like the sheet-like hemostatic material 10E, sufficient flexibility can be exhibited as long as the adhesive layer 11 is a partial layer.

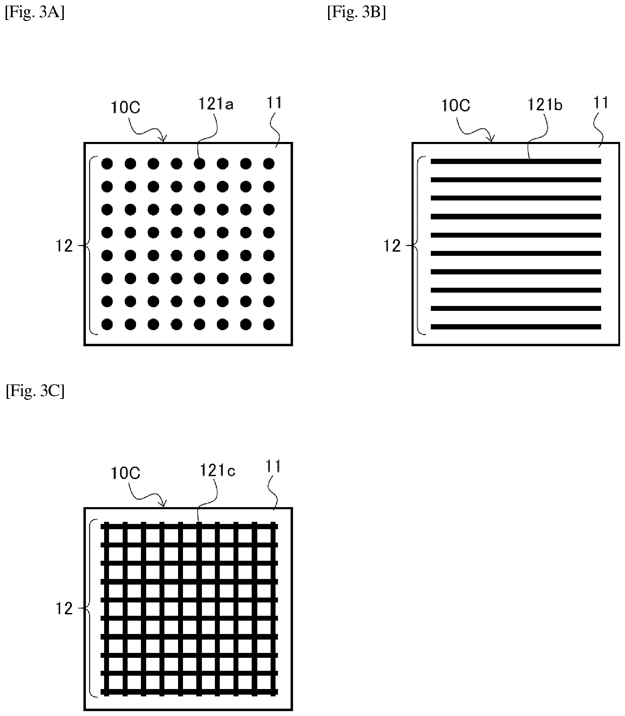

The specific shape of the partial covering layer 121, the partial adhesive layer 111, or the partial adhesive layer 141 is not particularly limited and may be configured as a partial layer (partial sheet) that is not a single sheet. For example, when the partial covering layer 121 is described as an example, it may include a dot-shaped partial covering layer 121a as shown in Fig. 3A, a linear partial covering layer 121b as shown in Fig. 3B, a shaded partial covering layer 121c as shown in Fig. 3C, and a shape other than these.

As described above, the partial layer or the partial sheet may be a member aggregate (see Fig. 3A or 3B) in which a plurality of planar members are arranged collectively in a dot-like shape or a line shape, a single planar member (see Fig. 3C) configured by forming a plurality of openings (or a plurality of through holes) like a shaded shape, or a combination of these (a configuration in which a plurality of single planar members having a plurality of openings collectively forms a member aggregate).

The partial covering layer 121 may be provided on the opposite surface so that the opposite surface of the adhesive layer 11 can be the aforementioned "non-adhesive surface", but it is preferable that the partial covering layer 121 is dispersedly arranged so as to extend over the entire opposite surface of the adhesive layer 11 as in the partial covering layer 121a to 121c shown in Figs. 3A to 3C. The dispersion arrangement of the partial covering layer 121 may be irregular (random), but it is more preferable that such arrangement is regular as shown in Figs. 3A to 3C. Note that the partial covering layer 121 shown in Fig. 2A configures one layer having a two-layer structure as in Fig. 1A, but as illustrated in Fig. 1B, the partial covering layer 121 may be provided as one layer of a multilayer structure of three or more layers.

Likewise, specific examples of the shape of the partial adhesive layer 111 or the partial adhesive layer 141 may include a dotted shape, a linear shape, a shaded shape, etc. similarly to the partial covering layer 121 described above. The partial adhesive layer 111 or the partial adhesive layer 141 may be provided on the adhesive surface so that it can serve as an "affixing surface" to be affixed to a bleeding site such as a biological tissue or the like, but similarly to the partial covering layer 121, it is preferable that the partial adhesive layer 111 or the partial adhesive layer 141 be dispersedly arranged so as to extend over the entire adhesive layer. The dispersion arrangement may be irregular but is preferably regular.

In the sheet-like hemostatic material 10A having a two-layer structure, the adhesive layer 1 1 and the covering layer 12 are integrally fixed to form one sheet. Similarly, in the sheet-like hemostatic material 10B having a structure of three or more layers, the adhesive layer 11, one or more intermediate layers 13, and the covering layer 12 may be integrally fixed to form one sheet.

In the sheet-like hemostatic material 10C, the covering layer 12 is configured by the partial covering layer 121. This partial covering layer 121 may be formed by punching out or cutting out a sheet to be the covering layer 12 into a predetermined shape and fixing it to the adhesive layer 11. Similarly, in the sheet-like hemostatic material 10D, the partial adhesive layer 111 may be formed by punching out or cutting out a sheet to be the adhesive layer 11 into a predetermined shape and fixing it to the covering layer 12. Further, since it is unnecessary to process the partial adhesive layer 141 into a sponge shape in the sheet-like hemostatic material 10E, a nonporous partial sheet formed from γ-PGA that is formed into a predetermined shape may be laminated to the adhesive layer 12 and then fixed, thereby to form the sheet-like hemostatic materials 10E.

A specific method of fixing these layers is not particularly limited, and a known method can be suitably used. If the covering layer 12 is a sponge-like sheet formed from a crosslinked collagen, as will be described later, a method of pressing and adhering the adhesive layer 11 and the covering layer 12 (one or more intermediate layers 13 as required) can be suitably used.

The adhesive layer 11 and the covering layer 12 in the sheet-like hemostatic materials 10A to 10E may have the same color, but preferably have different colors from each other. Thereby, when using the sheet-like hemostatic materials 10A to 10E, it can be easily visually confirmed which surface is the affixing surface (adhesive layer 11). The method of imparting color to the adhesive layer 11 and the covering layer 12 is not particularly limited, and a known method can be suitably used, but in general, a method in which a known coloring matter, dye, or pigment is added to the adhesive layer 11 or the covering layer 12 to color these layers can be mentioned. Specific examples of the coloring matter include legal coloring matters known in the field of medicines and the like, for example, Green No. 202, Purple No. 201, Blue No. 2, etc. Specific pigments include, for example, iron sesquioxide and the like.

The color may be applied to both the adhesive layer 11 and the covering layer 12 or only to one of the adhesive layer 11 and the covering layer 12. That is, both the adhesive layer 11 and the covering layer 12 may be colored by adding respective different coloring matters, or only one layer of the adhesive layer 11 and the covering layer 12 may be colored by adding a coloring matter or the like to only the adhesive layer 11 or only the covering layer 12. The method of adding the coloring matter or the like is also not particularly limited, and in the process of forming the adhesive layer 11 or the covering layer 12 (producing a sheet or a sponge-like porous body as these layers), the coloring matter or the like may be added to γ-PGA or the covering material. Alternatively, one of the adhesive layer 11 and the covering layer 12 may be colored with a coloring matter or the like after the sheet-like hemostatic materials 10A to 10E are manufactured. Further, the portion to which color is imparted is not particularly limited, as long as at least a part of the adhesive layer 11 or the covering layer 12 is given a color. Of course, all of the adhesive layer 11 or the covering layer 12 (entire layer) may be colored.

In the sheet-like hemostatic materials 10A to 10E, the weight ratio of the adhesive layer 11 and the covering layer 12 is not particularly limited, but typically the weight ratio of the adhesive layer 11 is preferably in the range of 10 to 90% by weight based on the total weight of each of the sheet-like hemostatic materials 10A to 10E. If the content of the adhesive layer 11 is less than 10% by weight, there is a possibility that adhesiveness by the adhesive layer 11 cannot be sufficiently exhibited although it depends on the configuration of the sheet-like hemostatic materials 10A to 10E. If the content of the adhesive layer 11 exceeds 90% by weight, there is a possibility that the effect of suppressing the adhesiveness of the adhesive layer 11 by the covering layer 12 cannot be sufficiently exhibited though it depends on the configuration of the sheet-like hemostatic materials 10A to 10E.

The size and the thickness of the sheet-like hemostatic materials 10A to 10E are also not particularly limited and can be appropriately set according to the use conditions and the like of the sheet-like hemostatic materials 10A to 10E. The sheet-like hemostatic materials 10A to 10E can be suitably used for laparoscopic surgery, but in this case, it is preferable that the sheet-like hemostatic materials 10A to 10E have a size of about several centimeters in the longitudinal and lateral directions in consideration of trocar passing property. In addition, when used for surgery other than laparoscopic surgery, or when used for emergency hemostasis of trauma or the like, it is possible to use the sheet-like hemostatic materials 10A to 10E each having a size that is larger than about several centimeters in the longitudinal and lateral directions as described above.

Depending on the type of the covering layer 12 or the intermediate layer 13, the sheet-like hemostatic materials 10A to 10E can be stored at room temperature. The room temperature herein used refers to a temperature within the range of 1 to 30°C defined by the Japanese Pharmacopoeia. The adhesive layer 11 showing the hemostatic performance in the sheet-like hemostatic materials 10A to 10E is formed from γ-PGA as described above, and γ-PGA can be stably stored even within the range of room temperature. Further, for example, if a crosslinked collagen is used as the covering layer 12, such a crosslinked collagen can also be stably stored within the range of room temperature. Therefore, if the sheet-like hemostatic material 10A is configured by the adhesive layer 11 and the covering layer 12 made of a crosslinked collagen, it can be stably stored at room temperature. Similarly, if the intermediate layer 13 is formed from a material that can be stored at room temperature, the sheet-like hemostatic material 10B can also be stably stored at room temperature. As a matter of course, it is also possible to stably store the sheet-like hemostatic material 10B at low temperature as needed.

Note that a fibrinogen-based sheet-like hemostatic material, for example, TachoSil (registered trademark), needs to be stored at a low temperature of 10°C or less. In addition, it is necessary to preserve an oxidized cellulose type sheet-like hemostatic material, for example, SURGICEL (registered trademark) NU-KNIT at 25°C or less. The ordinary temperature is within the range of 15 to 25°C, but in summer, the ordinary temperature usually exceeds 25°C in many cases. Therefore, the necessity of storage at 25°C or less means that storage at room temperature is substantially difficult.

### [Poly-γ-Glutamic Acid]

As described above, the adhesive layer 11 is a sponge-like sheet or a nonporous partial sheet formed from at least poly-γ-glutamic acid (y-PGA). Therefore, the adhesive layer 11 may be formed from γ-PGA only or may be a composition containing γ-PGA as a main component and other components.

The γ-PGA used as the adhesive layer 11 includes a polymer in which glutamic acids are peptide-bonded between the carboxyl group at the γ-position and the amino group at the α-position and are linearly linked, and a salt thereof. An example of a salt of γ-PGA is sodium polγ-γ-glutamate. The term "poly-γ-glutamic acid (y-PGA)" used in the present embodiment basically includes "poly-γ-glutamic acid and/or a salt thereof'.

The γ-PGA used as the adhesive layer 11 is not specifically particularly limited, but for example, one having a molecular weight within a specific range can be suitably used. As a preferable range of the molecular weight of γ-PGA, the weight average molecular weight Mw can be in the range of 200,000 to 13,000,000, more preferably within the range of 300,000 to 10,000,000. If the weight average molecular weight Mw of γ-PGA falls below the lower limit of 200,000 (if it is less than 200,000), good adhesiveness of the affixing surface may not be realized though it depends on the configuration of the adhesive layer 11 or the sheet-like hemostatic materials 10A to 10E. Although the upper limit of the weight average molecular weight Mw of γ-PGA is not particularly limited, it is normally 13,000,000 or less. This is because it is considered that it is difficult to produce a γ-PGA having a weight average molecular weight Mw exceeding 13,000,000 by a conventionally known manufacturing method.

As γ-PGA used as the adhesive layer 11, those having a kinematic viscosity v within a specific range can be suitably used. As the preferable kinematic viscosity ν of γ-PGA, the kinematic viscosity v at 37°C when dissolved in distilled water at a concentration of 0.05% by mass can be, for example, in the range of 2 cSt to 15 cSt, more preferably in the range of 2.5 cSt to 8 cSt.

If the kinematic viscosity v of γ-PGA falls below the lower limit of 2 cSt (if it is less than 2 cSt), there is a possibility that good adhesiveness of the affixing surface cannot be realized, although it depends on the configuration of the adhesive layer 11 or the sheet-like hemostatic materials 10A to 10E. Although the upper limit of the kinematic viscosity v of γ-PGA is not particularly limited, it is normally 15 cSt or less. This is because it is considered that it is difficult to produce a γ-PGA having a kinematic viscosity ν exceeding 15 cSt by a conventionally known manufacturing method.

In the γ-PGA used in the adhesive layer 11 in the present disclosure, it is preferable that the weight average molecular weight Mw is within the above-mentioned range, or its kinematic viscosity v is within the range of kinematic viscosity under the predetermined conditions, or both the weight average molecular weight Mw and the kinematic viscosity v are within the above ranges. It goes without saying that at least one of the weight average molecular weight Mw and the kinematic viscosity v of γ-PGA may deviate from the range described above depending on the configuration or the use conditions of the adhesive layer 11 or the sheet-like hemostatic materials 10A to 10E.

The method of obtaining such γ-PGA is not particularly limited. For example, γ-PGA satisfying at least one of the weight average molecular weight Mw and the kinematic viscosity v described above is commercially available as a food additive and the like, and the safety against the biological tissue (including the human body) is sufficiently secured. In addition, for example, γ-PGA can be easily produced by a known method using a microorganism derived from the genus Bacillus, such as Bacillus subtilis.

The adhesive layer 11 may be formed from substantially only γ-PGA, except for the case where it contains impurities unavoidable in technical common sense, but the adhesive layer 11 may be configured by a γ-PGA composition containing components other than γ-PGA. Components other than γ-PGA (other components) may include those that have biocompatible and biodegradable properties and do not interfere with the adhesiveness property of the adhesive layer 11 when incorporated into γ-PGA. Specifically, for example, other components include, but not limited thereto, saccharides such as monosaccharides (e.g. glucose), oligosaccharides, and polysaccharides; collagen; glycerin; polyethylene glycol; known additives (such as coloring matters described above); and the like. These other components may be used singly or in combination of two or more kinds thereof as appropriate.

The blending amount of the other component to the γ-PGA composition is not particularly limited as long as it does not interfere with the adhesiveness of the adhesive layer 11. Generally, it is sufficient that the amount of γ-PGA in the total amount of γ-PGA composition is such that it does not become less than 50% by weight. If the γ-PGA composition is composed only of γ-PGA and other components, the other components should be less than 50% by weight. When the γ-PGA composition contains saccharides, further flexibility can be imparted to the adhesive layer 11, and further improvement in adhesiveness property can be expected.

### [Covering Material]

As described above, the covering layer 12 is a sheet (preferably a sponge-like sheet) formed from at least one biodegradable material (covering material) other than γ-PGA. Therefore, the covering layer 12 may be formed from one kind of covering material or may be formed from two or more kinds of covering materials. In addition to the covering material, other components that can be blended may be contained.

The covering material may be one that exhibits good biocompatibility when introduced into the biological tissue together with γ-PGA and is decomposed and absorbed after a certain period of time. Normally, polymers having biocompatibility and biodegradability can be mentioned as the covering material. As such a polymer, for example, collagen, polylactic acid, polyglycolic acid, and the like can be mentioned. Of these, collagen (Col) is particularly preferable. After being introduced into the biological tissue, collagen has almost no possibility of giving an undesirable influence on the biological tissue with excellent degradability in vivo and does not show substantially adhesiveness as compared with γ-PGA.

The specific configuration of collagen is not particularly limited, but preferably collagen which has been treated so that it can be dissolved in a known solvent. Specifically, for example, solubilized collagen such as enzyme-solubilized collagen, acid-solubilized collagen, alkali-solubilized collagen, and neutral-solubilized collagen can be mentioned. Among them, preferred collagen includes acid-solubilized collagen from the viewpoint of handleability. From the viewpoint of further reducing the possibility of giving an unfavorable effect to the biological tissue, atelocollagen which has been subjected to removal treatment of telopeptide, an antigenic determinant, can be preferably used.

The origin of collagen is not particularly limited, and includes cattle, pig, birds, fish, rabbit, sheep, rat, human, and the like. The part of the animal species to be extracted for collagen is also not particularly limited, and examples thereof include skin, tendon, bone, cartilage and organ, and the like. From the viewpoint of availability, those derived from porcine skin can be preferably used among these. Furthermore, the type of collagen is also not particularly limited, but it is exemplified by type I, type Π, and type III, etc. Among them, types I and III can be preferably used from the viewpoint of handleability.

A covering material such as collagen may be used for the covering layer 12 as it is, but such a covering material is preferably subjected to a crosslinking treatment. That is, the covering layer 12 is preferably formed from a crosslinked collagen (C-Col). As a result, the strength of the covering layer 12 can be improved or stabilized, and the decomposition time in vivo can be controlled by adjusting the degree of crosslinking. The method of crosslinking the covering material is not particularly limited, and examples thereof include a chemical crosslinking method, γ-ray irradiation, ultraviolet irradiation, electron beam irradiation, plasma irradiation, thermal dehydration crosslinking treatment, and the like. When the covering material contains at least collagen, a thermal dehydration crosslinking treatment can be preferably mentioned among these crosslinking methods.

In the thermal dehydration crosslinking treatment, collagen is first formed into a sheet and then dried in the air. Thereafter, the sheet is placed in a vacuum dry oven and kept at a predetermined temperature for a predetermined time under reduced pressure. This makes it possible to crosslink collagen. In the thermal dehydration crosslinking treatment, the degree of crosslinking can be well adjusted by adjusting at least one of the crosslinking temperature and the crosslinking time.

When the covering layer 12 contains not only one or more types of biodegradable materials (covering materials) but also other components that can be blended into the biodegradable material, that is, when the covering layer 12 is formed from a covering material composition, other components can be blended within a range that does not interfere with the physical properties of the covering layer 12, similarly to the γ-PGA composition described above. In general, it is sufficient that the blending amount is such that the covering material does not become less than 50% by weight based on the total amount of the covering material composition. If the covering material composition consists only of one or more covering materials and other components, the content of the other components may be less than 50% by weight. In addition, as other components, known additives (such as the above-mentioned coloring matter etc.) and the like can be mentioned, but there is no particular limitation.

### [Method of Manufacturing Sheet-Like Hemostatic Material]

The method of manufacturing the sheet-like hemostatic material according to the present disclosure is not particularly limited, and any method can be used as long as it is a method capable of manufacturing a sheet-like hemostatic material having sufficient flexibility by forming an adhesive layer configured by at least γ-PGA. The sheet-like hemostatic materials 10A to 10E according to the present disclosure are provided with the adhesive layer 11 and the covering layer 12 as described above and may also include the intermediate layer 13. Therefore, as a representative manufacturing method, there may be mentioned preferably a method in which an adhesive layer 11 which is a sponge-like sheet formed from γ-PGA or a nonporous partial sheet, a covering layer 12 which is a sheet made of a covering material, and an intermediate layer 13 interposed therebetween as needed may be respectively laminated to be integrally fixed as one sheet. Therefore, these sheets for the adhesive layer 11, the covering layer 12, and the intermediate layer 13 may be formed simultaneously, or an arbitrary sheet may be first formed and then another sheet may be formed.

As a typical manufacturing method of the sheet-like hemostatic material when both the adhesive layer 11 and the covering layer 12 are sponge-like sheets, for example, there can be mentioned a manufacturing method including a step of laminating a first sponge-like porous body formed from at least γ-PGA or a first sponge-like sheet obtained by applying pressure to the first sponge-like porous body and a second sponge-like porous body formed from a covering material or a second sponge-like sheet obtained by applying pressure to the second sponge-like porous body to form a laminate (lamination step), a step of laminating the first sponge-like porous body or the first sponge-like sheet and the second sponge-like porous body or the second sponge-like sheet to form a laminate (lamination step), and a step of bonding or pressure-bonding the laminate to form a sheet-like hemostatic material 10A (or a sheet-like hemostatic material 10B) including the adhesive layer 11 and the covering layer 12 (integration step).

In addition, the method of manufacturing a sheet-like hemostatic material may include a step of forming a sponge-like porous body or a sheet used in the lamination step. In other words, in the lamination step of the present disclosure, a first sponge-like sheet or a second sponge-like sheet produced in advance (or a commercially available one) may be used, or a first sponge-like porous body or a second sponge-like porous body produced in advance may be used, or a sponge-like porous body or a sponge-like sheet may be produced from the raw material (γ-PGA or covering material).

Specifically, the method of manufacturing a sheet-like hemostatic material according to the present disclosure may include at least one of a step of freeze-drying at least γ-PGA to form a first sponge-like porous body (first sponge-like porous body forming step), a step of forming a first sponge-like sheet by applying pressure to the first sponge-like porous body (first sponge-like sheet forming step), a step of freeze-drying a covering material to form a second sponge-like porous body (second sponge-like porous body forming step), and a step of applying pressure to the second sponge-like porous body to form a second sponge-like sheet (second sponge-like sheet forming step).

In the first sponge-like porous body forming step or the second sponge-like porous body forming step, γ-PGA or a covering material may be molded into a sponge-like porous body using the above-mentioned various drying methods. In the case where the covering layer 12 contains collagen, the second sponge-like porous body may be formed by a vacuum freeze-drying method. If the collagen of the covering layer 12 is a crosslinked collagen, the crosslinking treatment may be carried out on the sponge-like porous body in a state before or after the sponge formation. Alternatively, the sponge-like porous body may be compressed to form a sponge-like sheet, and this sponge-like sheet may be subjected to the crosslinking treatment. Further, as described above, the first sponge-like porous body or the second sponge-like porous body may be formed into a sheet shape by pressurization using a press or the like.

In the lamination step and the integration step, known lamination methods, bonding methods, and pressure-bonding methods may be used. For example, as a pressure-bonding method, a known press machine may be used. As a lamination method, not only a manual lamination method by an operator but also a method of automatically laminating by a known robot or the like can be suitably used.

In the manufacturing method of a sheet-like hemostatic material according to the present disclosure, the integration step cannot be performed until after the lamination step, but in each of the steps that can be performed before the lamination step, that is, the order of the first sponge-like porous body forming step, the second sponge-like porous body forming step, the first sponge-like sheet forming step, the second sponge-like sheet forming step, and the lamination step is not particularly limited. In addition, the method of manufacturing a sheet-like hemostatic material according to the present disclosure should include at least the lamination step and the integration step, and if necessary, the first sponge-like porous body forming step, the second sponge-like porous body formation step, the first sponge-like sheet forming step, and the second sponge-like sheet forming step may be included, but steps other than these steps may be included. For example, a step of sterilizing after the integration step may be included. Furthermore, in the method of manufacturing a sheet-like hemostatic material according to the present disclosure, any plural steps among these steps may be performed concurrently in parallel.

As a preferable example of the method of manufacturing a sheet-like hemostatic material, for example, there can be mentioned a method in which a pressure is first applied to a second sponge-like porous body to prepare a second sponge-like sheet, and the obtained second sponge-like sheet is then laminated and pressure-bonded on the first sponge-like porous body. In this manufacturing method, the lamination step is performed after the second sponge-like sheet forming step, and thereafter the first sponge-like sheet forming step is carried out. By this method, it is possible to efficiently produce a sheet-like hemostatic material 10A having a two-layer structure shown in Fig. 1A.

In the case where the adhesive layer 11 or the covering layer 12 is a partial layer like the sheet-like hemostatic material 10C, 10D or 10E, as a method of manufacturing the sheet-like hemostatic material, a step of punching out or cutting out a sheet to be the adhesive layer 11 or covering layer 12 into a predetermined shape (partial layer forming step) may be performed before the lamination step. In other words, the method of manufacturing a sheet-like hemostatic material according to the present disclosure may include a partial layer forming step in addition to the lamination step and the integration step. Alternatively, these partial layers may be formed as a partial sheet from the beginning, rather than being formed as a partial sheet by processing a single sheet.

As described above, in the method of manufacturing a sheet-like hemostatic material according to the present disclosure, in order to form the adhesive layer 11, it is possible not only to use the first sponge-like porous body which is formed from at least γ-PGA or the sponge-like sheet obtained by applying pressure to the first sponge-like porous body, but also to use the first partial sheet which is formed from at least γ-PGA and is not a single sheet. In this way, a layered member made of γ-PGA prepared for forming the adhesive layer 11 is referred to as a first layered body for convenience. Note that the first partial sheet as the first layered body may be in the form of a sponge shape or may be in the form of a nonporous shape which is not sponge-like.

Similarly, in order to form the covering layer 12, not only the second sponge-like porous body formed from a biodegradable material described above or the second sponge-like sheet obtained by applying pressure to the second sponge-like porous body, but also the second partial sheet which is formed from a biodegradable material and is not a single sheet can be used. In this way, a layered member made of a biodegradable material prepared for forming the covering layer 12 is referred to as a second layered body for convenience.

Therefore, as a representative example of the method of manufacturing a sheet-like hemostatic material according to the present disclosure, there can be mentioned a configuration including a step of laminating a first layered body formed from at least γ-PGA and a second layered body formed from at least one biodegradable material other than γ-PGA to form a laminate; and a step of bonding or pressure-bonding the laminate to form a sheet-like hemostatic material including an adhesive layer formed from at least the γ-PGA and a covering layer formed from the biodegradable material.

As described above, according to the present disclosure, the affixing surface of the sheet-like hemostatic material is an adhesive layer formed from poly-γ-glutamic acid (y-PGA), and the non-affixing surface is a covering layer formed from a biodegradable material (covering material) other than γ-PGA. Therefore, γ-PGA of the adhesive layer can realize good adhesiveness to biological tissues and good hemostatic performance without containing animal materials such as fibrinogen, and by using the covering material of the covering layer, it is possible to effectively suppress unintentional adhesiveness of the sheet-like hemostatic material to the tissues other than the affixing site, or to surgical instruments or the like.

In addition, since the adhesive layer is a sponge-like sheet or a nonporous partial layer, it has good flexibility and the covering layer also has flexibility. This makes it possible to exhibit good flexibility as the sheet-like hemostatic material. Therefore, even if the sheet-like hemostatic material is rounded or bent, breakage of the adhesive layer can be effectively suppressed. Further, by forming one of the adhesive layer and the covering layer as a base material layer composed of a single sheet and by forming the other as a partial layer, the flexibility of the sheet-like hemostatic material can be improved.

Furthermore, since γ-PGA has good adhesiveness without accompanying biochemical reactions, it is possible to reapply γ-PGA to a biological tissue when compared to a conventional sheet-like hemostatic material. In addition, since the adhesive layer is formed from γ-PGA, by appropriately selecting the covering material to be the covering layer, it is possible to store the sheet-like hemostatic material of the present invention for a longer period of time at room temperature as compared with the conventional sheet-like hemostatic material.

### (Embodiment 2)

Another sheet-like hemostatic material according to the present disclosure has a configuration including an adhesive layer which is formed from at least poly-γ-glutamic acid and to which a saccharide is added. Hereinafter, representative embodiments of the present disclosure will be specifically described.

### [Sheet-Like Hemostatic Material]

As described above, the sheet-like hemostatic material according to the present disclosure may include, as an adhesive layer, a γ-PGA sheet which is formed from poly-γ-glutamic acid (γ-PGA) as a main component and to which a saccharide has been added (a saccharide added γ-PGA sheet). By adding a saccharide to γ-PGA to form a sheet, flexibility can be imparted to the γ-PGA sheet without deteriorating adhesiveness to biological tissue (see examples described later).

The sheet-like hemostatic material may be a monolayer sheet formed from only a saccharide-added γ-PGA sheet but may be a multilayer structure sheet of two or more layers in which other layers are laminated as necessary. As the other layer, it can include, for example, but not limited to, a reinforcing layer for improving the strength of the sheet-like hemostatic material, a covering layer which covers one side of a saccharide-added γ-PGA sheet so that only the other side is an adhesive surface (affixing surface), and the like.

The other layers may have biocompatibility and biodegradability similarly to γ-PGA, and the specific material thereof is not particularly limited. As a representative other layer, for example, a crosslinked collagen (C-Col) can be mentioned as a covering layer. A method of laminating other layers is also not particularly limited, and a known method can be suitably used.

The size and the thickness of the sheet-like hemostatic material according to the present disclosure are also not particularly limited and can be appropriately set in accordance with the use conditions of the sheet-like hemostatic material and the like. Although the sheet-like hemostatic material according to the present disclosure can be suitably used for laparoscopic surgery, the sheet-like hemostatic material is suitable in this case if it has a size of about several centimeters in the longitudinal and lateral directions in consideration of the trocar passing ability. In addition, when used for surgery other than laparoscopic surgery, or when used at the time of emergency hemostasis of trauma or the like, it is possible to use the sheet-like hemostatic material that is larger than about several centimeters in length and width as described above.

The sheet-like hemostatic material according to the present disclosure can be stored at room temperature because the adhesive layer is formed from a saccharide-added γ-PGA sheet. The room temperature herein used is a temperature within a range of 1 to 30°C defined as appropriate by the Japanese Pharmacopoeia. The adhesive layer (saccharide-added γ-PGA sheet) showing hemostatic performance in the sheet-like hemostatic material contains γ-PGA as a main component and a saccharide as an auxiliary component, but both γ-PGA and saccharide can be stably stored even within the range of room temperature. As a matter of course, it is also possible to stably store the sheet-like hemostatic material at a low temperature if necessary.

Note that a fibrinogen-based sheet-like hemostatic material, for example, TachoSil (registered trademark), needs to be stored at a low temperature of 10°C or less. In addition, it is necessary to preserve oxidized cellulose type sheet-like hemostatic material, for example, SURGICEL (registered trademark) NU-KNIT at 25°C or less. The ordinary temperature is within the range of 15 to 25°C but in summer, the air temperature usually exceeds 25°C in many cases. Therefore, preservation at 25°C or less means that preservation at room temperature is substantially difficult.

The method of manufacturing a sheet-like hemostatic material according to the present disclosure is not particularly limited and may be a manufacturing method including a step of forming an adhesive layer by adding at least a saccharide to γ-PGA to mold it into a sheet (to form a saccharide-added γ-PGA sheet). In the case of providing other layers besides the adhesive layer, the saccharide-added γ-PGA sheet to be an adhesive layer and the sheet to be another layer may be laminated and fixed by a known method such as pressure-bonding. The method of adding a saccharide to γ-PGA and the method of molding a saccharide-added γ-PGA into a sheet form are also not particularly limited, and known methods can be suitably used.

Further, the sheet-like hemostatic material according to the present disclosure may include at least an adhesive layer, which is a saccharide-added γ-PGA sheet obtained by molding a γ-PGA composition including at least γ-PGA and a saccharide. Therefore, it is also possible to say that the method of manufacturing a sheet-like hemostatic material according to the present disclosure is a method of preparing a γ-PGA composition and molding it into a sheet. That is, in the present disclosure, since the sheet-like hemostatic material can be easily manufactured by molding the γ-PGA composition into a sheet shape, complicated manufacturing processes are not required. Further, for example, if the γ-PGA composition is formed into a sheet by a casting method, it becomes possible to control the thickness of the saccharide-added γ-PGA sheet only by adjusting the liquid amount of a liquid γ-PGA composition.

### [Poly-γ-Glutamic Acid]

The saccharide-added γ-PGA sheet serving as an adhesive layer may be one having γ-PGA as a main component and a saccharide as an auxiliary component. The γ-PGA used for the adhesive layer includes a polymer in which glutamic acids are linked linearly by forming peptide bonds between the carboxyl group at the γ-position and the amino group at the α-position, and a salt thereof. An example of the salt of γ-PGA is sodium poly-γ-glutamate. The term "poly-γ-glutamic acid (y-PGA)" used in the present embodiment basically includes "poly-γ-glutamic acid and/or a salt thereof".

The γ-PGA used as the adhesive layer is not particularly limited, but includes specifically, for example, a γ-PGA having a molecular weight within a specific range can be suitably used. As a preferable range of the molecular weight of γ-PGA, the weight average molecular weight Mw can be in the range of 200,000 to 13,000,000, more preferably within the range of 300,000 to 10,000,000. If the weight average molecular weight Mw of γ-PGA falls below the lower limit of 200,000 (if it is less than 200,000), it may be impossible to realize good adhesiveness on the affixing surface although adhesiveness depends on the configuration of the adhesive layer or the sheet-like hemostatic material. The upper limit of the weight average molecular weight Mw of γ-PGA is not particularly limited but is normally 13,000,000 or less. This is because it is considered that it is difficult to produce γ-PGA having a weight average molecular weight Mw exceeding 13,000,000 by a conventionally known manufacturing method.

As the γ-PGA used as an adhesive layer, γ-PGA having a kinematic viscosity v within a specific range can be suitably used. As the preferable kinematic viscosity v of γ-PGA, the kinematic viscosity v at 37°C when γ-PGA is dissolved in distilled water at a concentration of 0.05% by mass can be in the range of, for example, 2 cSt to 15 cSt, preferably in the range of 2.5 cSt to 8 cSt. If the kinematic viscosity v of γ-PGA falls below the lower limit of 2 cSt (if less than 2 cSt), it may not be possible to achieve good adhesiveness on the affixing surface, though it depends on the configuration of the adhesive layer or the sheet-like hemostatic material. Although the upper limit of the kinematic viscosity v of γ-PGA is not particularly limited, it is normally 15 cSt or less. This is because it is considered that it is difficult to manufacture γ-PGA having a kinematic viscosity v exceeding 15 cSt by a conventionally known manufacturing method.

The γ-PGA used in the adhesive layer in the present disclosure may have a weight average molecular weight Mw within the above range or its kinematic viscosity v may be within the range of kinematic viscosity under the predetermined condition, or both the weight average molecular weight Mw and the kinematic viscosity v may be within the above-mentioned ranges. It goes without saying that at least one of the weight average molecular weight Mw and the kinematic viscosity ν of γ-PGA may deviate from the range described above, depending on the configuration of the adhesive layer or the sheet-like hemostatic material or the use conditions and the like.

The method of obtaining such γ-PGA is not particularly limited. For example, γ-PGA satisfying at least one of the weight average molecular weight Mw and the kinematic viscosity ν is commercially available as a food additive and the like, and the safety to the biological tissue (including the human body) is sufficiently secured. In addition, for example, γ-PGA can be easily manufactured by a known method using a microorganism derived from the genus Bacillus, such as Bacillus subtilis.

### [Saccharides and Other Components]

The saccharide contained in the saccharide-added γ-PGA sheet may be a polyhydric alcohol (monosaccharide) having a carbonyl group, or a polymer thereof (oligosaccharide or polysaccharide). Specific types of monosaccharide, oligosaccharide, and polysaccharide are not particularly limited, and known compounds can be suitably used. In particular, in the present disclosure, since the sheet-like hemostatic material is introduced into the biological tissue and affixed to the biological tissue or the like, the saccharide can be added to γ-PGA as long as it does not have a property incompatible with the biological tissue.

Specific examples of the monosaccharide include hexoses (6-carbon sugar) such as glucose, galactose, fructose, and mannose; pentoses (5-carbon sugar) such as arabinose, xylose, and ribose; amino sugars or derivatives thereof, such as glucosamine, galactosamine, N-acetyl-glucosamine, N-acetylgalactosamine, and sialic acid; sugar alcohols such as sorbitol and xylitol; and the like.

Examples of the oligosaccharide include disaccharides such as trehalose, maltose, sucrose, lactose, and melibiose; trisaccharides such as raffinose; and tetrasaccharides such as stachyose.

Examples of the polysaccharide include glucans such as amylose, amylopectin, dextran, dextrin, and icodextrin; glycosaminoglycans such as hyaluronic acid, alginic acid, heparan sulfate, chondroitin sulfate, chondroitin, dermatan sulfate, keratosulfate, and heparin; dietary fiber type non-glucan polysaccharides such as alginic acid and salts thereof, inulin, carboxymethylcellulose, pectin, chitin, chitosan, carrageenan, and fucoidan; and the like.

Only one kind of these monosaccharides, oligosaccharides, and polysaccharides may be added to the saccharide-added γ-PGA sheet, or plural types thereof may be appropriately combined and added to the saccharide-added γ-PGA sheet. The plural kinds mentioned here may be a combination of monosaccharides, a combination of oligosaccharides, or a combination of polysaccharides, or a combination of monosaccharide and oligosaccharide, a combination of monosaccharide and polysaccharide, or a combination of oligosaccharide and polysaccharide.

The amount of these saccharides to be added is not particularly limited, but generally such amount may be within the range of 10 to 90% by weight based on the total weight of the saccharide-added γ-PGA sheet. If the addition amount of the saccharide is less than 10% by weight, flexibility of the saccharide-added γ-PGA sheet cannot be sufficiently obtained, so that the sheet may be warped or broken, depending on the kind of saccharides or the conditions (molecular weight etc.) of γ-PGA. In addition, if the addition amount of the saccharide exceeds 90% by weight, the adhesiveness (function as an adhesive layer) of the saccharide-added γ-PGA sheet may not be sufficiently obtained, though it depends on the kind of saccharides or the conditions (molecular weight etc.) of γ-PGA.

In addition, the saccharide-added γ-PGA sheet may contain other components besides γ-PGA and saccharides. The components other than γ-PGA (other components) may include those which have biocompatibility and biodegradability, do not hinder the adhesiveness of the saccharide-added γ-PGA sheet when incorporated into γ-PGA, and do not interfere with the flexibility of the saccharide-added γ-PGA sheet by the addition of saccharides. Specific examples of the other components can include, but not particularly limited to, collagen, glycerin, polyethylene glycol, known additives (coloring materials etc.), and the like. These other components may be used singly or in combination of two or more kinds thereof as appropriate. The addition amount of the other components may be within a range that does not hinder the adhesiveness and flexibility of the saccharide-added γ-PGA sheet.

From the viewpoint of improving the handleability of the sheet-like hemostatic material according to the present disclosure, collagen is particularly preferable among the other components described above. By adding collagen to the saccharide-added γ-PGA sheet, breakage can be effectively suppressed even if the saccharide-added γ-PGA sheet (adhesive layer) is deformed by being rolled or bent. In addition, the saccharide-added γ-PGA sheet (adhesive layer) to which collagen is added is easily restored to its original shape even if the sheet is deformed.

Therefore, if the sheet-like hemostatic material according to the present disclosure is provided with a saccharide-added γ-PGA sheet as an adhesive layer to which collagen is added, for example, even if a planar sheet-like hemostatic material is rolled and inserted into a trocar during laparoscopic surgery, the sheet-like hemostatic material is easily restored to its original planar shape after passing through the trocar. Therefore, since the operation of deforming the sheet-like hemostatic material into a desired shape and then applying a physical force to restore the shape to its original shape can be minimized, handleability of the sheet-like hemostatic material can be remarkably improved.

In the sheet-like hemostatic material according to the present disclosure, when attention is focused on improving the handleability, a γ-PGA sheet to which only collagen is added and no saccharide is added (collagen-added γ-PGA sheet) may be provided as an adhesive layer. In other words, the present disclosure also includes a sheet-like hemostatic material including a sheet as an adhesive layer which is formed from at least poly-γ-glutamic acid and to which collagen has been further added.

Here, since the method of manufacturing the collagen-added γ-PGA sheet and the method of manufacturing the sheet-like hemostatic material including the collagen-added γ-PGA sheet serving as the adhesive layer are the same as the method of manufacturing the saccharide-added γ-PGA sheet, a detailed description thereof will be omitted. For example, although the addition amount of collagen is not particularly limited, the amount of collagen to be added may be within the range of 10 to 90% by weight based on the total weight of the collagen-added γ-PGA sheet, similarly to the addition amount of the saccharide in the saccharide-added γ-PGA sheet.

As described above, in the sheet-like hemostatic material according to the present disclosure, the adhesive layer is configured by a saccharide-added γ-PGA sheet which is an adhesive layer formed from γ-PGA as a main component and added with a saccharide. A γ-PGA sheet consisting essentially of γ-PGA alone does not have flexibility and breaks when it is intended to be deformed, but a γ-PGA sheet containing a saccharide can exhibit good flexibility.

Therefore, γ-PGA of the adhesive layer can realize good adhesiveness to biological tissue and good hemostatic performance without containing animal materials such as fibrinogen, and can also effectively suppress breakage of the adhesive layer even if the sheet-like hemostatic material is rolled or bent. Furthermore, since γ-PGA has good adhesiveness without accompanying biochemical reactions, it is possible to reapply to the biological tissue as compared to the conventional sheet-like hemostatic material. In addition, since the adhesive layer is formed from γ-PGA, the sheet-like hemostatic material can be stored for a longer time at room temperature as compared with the conventional sheet-like hemostatic material.

Further, in the sheet-like hemostatic material according to the present disclosure, the adhesive layer may be configured by a collagen-added γ-PGA sheet formed from γ-PGA as a main component and added with collagen. This configuration makes it possible to improve the handleability of the sheet-like hemostatic material. Of course, it goes without saying that the adhesive layer of the sheet-like hemostatic material according to the present disclosure may be a γ-PGA sheet which contains γ-PGA as a main component and to which both of a saccharide and collagen are added.

### Examples

Although the present disclosure will be described more specifically based on examples and comparative examples, the present disclosure is not limited thereto. Those skilled in the art can make various changes, modifications, and alterations without departing from the scope of the present disclosure.

First, the sheet-like hemostatic material having the configuration described in Embodiment 1 will be specifically described based on Examples 1 to 3 and Comparative Examples 1 to 3. The sheet-like hemostatic materials in the following Examples 1 to 3 and Comparative Examples 1 to 3 were evaluated in the following manner.

### (Evaluation of Hemostatic performance and Reapplication)

The abdomen of a 40-week-old male rabbit (body weight: 3.0 kg) was incised under continuous anesthesia to expose the liver and the surface of the liver was incised to cause bleeding. Then, the sheet-like hemostatic material of each of Examples 1 to 3 or Comparative Examples 1 to 3 was affixed to the bleeding site. The condition of bleeding after affixing the hemostatic material was confirmed to evaluate hemostatic performance. In addition, evaluation was also performed as to whether or not the reapplication of the sheet-like hemostatic material of each of Examples 1 to 3 or Comparative Examples 1 to 3 to the bleeding site of the liver is possible.

### (Evaluation of Trocar Passing Property)

Each of the sheet-like hemostatic materials of Examples 1 to 3 or Comparative Examples 1 to 3 was rolled into a roll and evaluated as to whether or not the sheet-like hemostatic material could pass through a trocar having a diameter of 5 mm (product name: Step System Versa Step 5 MM, manufactured by Covidien Japan Co., Ltd,).

### (Example 1)

A 1% by weight aqueous collagen solution was prepared, filled in a rectangular metal container in a predetermined amount, and frozen at -20°C for about 12 hours. The obtained frozen product was freeze-dried in a freeze dryer (product name: FDU-2100, manufactured by Tokyo Rikakikai Co., Ltd.) under reduced pressure (about 13 Pa (1 Torr) or less) for about 24 hours, and compressed at a pressure of 980 N/cm² (100 kgf/cm²) by a 15-ton press machine, manufactured by Masada Manufacturing Co., Ltd.). The obtained freeze-dried product was subjected to thermal dehydration crosslinking under reduced pressure (about 13 Pa (1 Torr) or less) for about 24 hours in a vacuum dry oven (product name: VOS-300VD, manufactured by Tokyo Rikakikai Co., Ltd.). Thus, a sponge-like sheet of crosslinked collagen (C-Col) was obtained.

Next, a 1% by weight aqueous γ-PGA solution was prepared, filled in a rectangular metal container in a predetermined amount, and frozen at -20°C for about 12 hours to obtain a sponge-like porous body. Thereafter, the sponge-like porous body was freeze-dried under reduced pressure for about 24 hours, then a crosslinked collagen sponge-like sheet was laminated and pressure-bonded on the sponge-like porous body. Thus, the γ-PGA sponge-like sheet serving as an adhesive layer and the crosslinked collagen sponge-like sheet serving as a covering layer were pressure-bonded to obtain a sheet-like hemostatic material according to Example 1.

The weight of this sheet-like hemostatic material was 90 mg having a size of 2.5 cm × 2.5 cm, and the weight ratio of the crosslinked collagen sponge-like sheet to the γ-PGA sponge-like sheet was 30 : 70. Using this sheet-like hemostatic material, the hemostatic performance and reapplication performance were evaluated as described above. The results are shown in Table 1.

### (Example 2)

A sheet-like hemostatic material according to Example 2 was obtained in the same manner as in Example 1, except that the filling amount of the 1% by weight aqueous collagen solution and the 1% by weight aqueous γ-PGA solution into the metal container was changed so that the weight ratio of the crosslinked collagen sponge-like sheet to the γ-PGA sponge-like sheet was 25 : 75. The sheet-like hemostatic material having a size of 2.5 cm × 2.5 cm was 80 mg in weight, and the weight ratio of the crosslinked collagen sponge-like sheet to the γ-PGA sponge-like sheet was 25 : 75 as described above. Using this sheet-like hemostatic material, the hemostatic performance and reapplication performance were evaluated as described above. The results are shown in Table 1.

### (Comparative Example 1)

Using 123 mg of SURGICEL NU-KNIT (registered trademark; manufactured by Johnson & Johnson) of 2.5 cm × 2.5 cm as the sheet-like hemostatic material according to Comparative Example 1, the hemostatic performance and reapplication performance as described above were evaluated as described above. The results are shown in Table 1.

**[Table 1]**

| | Example 1 | Example 2 | Comparative Example 1 |
|---|---|---|---|
| Sheet-like hemostatic material | C-Col: γ-PGA= 30:70 | C-Col : γ-PGA= 25:75 | SURGICEL® NU-KNIT |
| Size [cm] | **2.5 × 2.5** | **2.5 × 25** | **2.5 × 2.5** |
| Weight [mg] | **90** | **80** | **123** |
| Hemostasis performance | Good | Good | Good |
| Reapplication performance | Good | Good | Poor |

### (Example 3)

As the sheet-like hemostatic material according to Example 3, a sheet having a size of 3.0 cm × 2.5 cm was prepared in the same manner as in Example 1. Using this sheet-like hemostatic material, the trocar passing property was evaluated as described above. The results are shown in Table 2.

### (Comparative Example 2)

Using a TachoSil (registered trademark, CSL Behring Co., Ltd.) of 3.0 cm × 2.5 cm as the sheet-like hemostatic material according to Comparative Example 2, the trocar passing property was evaluated as described above. The results are shown in Table 2.

### (Comparative Example 3)

A 1% by weight aqueous γ-PGA solution was prepared, and a γ-PGA sheet of 3.0 cm × 2.5 cm was produced by a casting method to obtain a sheet-like hemostatic material according to Comparative Example 3. This γ-PGA sheet was used to evaluate the trocar passing property as described above. The results are shown in Table 2.

**[Table 2]**

| | Example 3 | Comparative Example 2 | Comparative Example 3 |
|---|---|---|---|
| Sheet-like hemostatic material | C-Col:γ-PGA= 30:70 | TachoSil® | γ-PGA monolayer, non-sponge-like |
| Size [cm] | 3.0 × 2.5 | 3.0 × 2.5 | 3.0 × 2.5 |
| Trocar passing property | Good | Poor | Poor |
| | The sheet in a roll form easily passes through the trocar. | If pushed in a crushed roll shape, the sheet passes through the trocar but breaks. | The sheet cracks and cannot be rolled into a roll shape. |

(Comparison of Examples 1 to 3 and Comparative Examples 1 to 3)

In the case of the sheet-like hemostatic materials according to Examples 1 and 2, they could exhibit good adhesiveness and hemostatic performance by merely affixing the hemostatic material to the biological tissue and lightly pressing it. In addition, it was possible to peel off the hemostatic material after once being affixed to the biological tissue and then affix it to the biological tissue again. On the other hand, in the SURGICEL NU-KNIT according to Comparative Example 1, it was possible to exhibit the hemostatic performance by pressing the SURGICEL NU-KNIT for 3 minutes after being affixed to the biological tissue, but unlike the sheet-like hemostatic material according to the present disclosure, reapplication of the hemostatic materials was impossible.

Further, in the case of the sheet-like hemostatic material according to Example 3, the hemostatic material can easily pass through the trocar by making it into a roll shape. However, in the case of TachoSil (Comparative Example 2), TachoSil could barely pass through the trocar by bending it into a roll shape, but TachoSil was damaged. In addition, since the γ-PGA sheet (Comparative Example 3) was a γ-PGA monolayer which is not sponge-like, such γ-PGA sheet was not flexible and cracked when the sheet is bent, resulting in failure to mold it into a roll shape.

As described above, the sheet-like hemostatic material according to the present disclosure is superior to the conventional one in terms of hemostatic performance without containing fibrinogen and can realize good flexibility and handleability.

Next, the sheet-like hemostatic material having the configuration described in Embodiment 2 will be specifically described based on Examples 4 to 8 and Comparative Examples 4 to 5. In the following Examples 4 to 8 and Comparative Examples 4 to 5, the sheet-like hemostatic materials were evaluated in the following manner.

### (Evaluation of Sheet State)

With respect to the sheet-like hemostatic materials (saccharide-added γ-PGA sheets or γ-PGA sheets) produced in Examples 4 to 8 or Comparative Examples 4 to 5, warpage and the like were visually observed, and folding was attempted on the sheet-like hemostatic materials.

### (Evaluation of Trocar Passing Property)

Sheet-like hemostatic materials (saccharide-added γ-PGA sheets or γ-PGA sheets) of 3.0 cm × 2.5 cm were prepared in Examples 4 to 8 or Comparative Examples 4 to 5, rolled into a roll shape, and evaluated as to whether or not they could pass through a trocar of 5 mm in diameter (product name: Step System Versa Step 5 MM, manufactured by Covidien Japan Co., Ltd).

### (Example 4)

A γ-PGA-saccharide solution (1% γ-PGA 1% Glu solution) of 1% by weight γ-PGA and 1% by weight glucose was prepared by adding glucose (Glu) to 1% by weight γ-PGA solution. This γ-PGA-saccharide solution was developed on a stainless steel plate as a base material and dried (casting method) to obtain a saccharide-added γ-PGA sheet (γ-PGA 50% by weight, Glu 50% by weight) as a sheet-like hemostatic material according to Example 4.

With respect to the saccharide-added γ-PGA sheet, the sheet state and the trocar passing property were respectively evaluated by the above-mentioned methods. The results of the sheet state are shown in Table 1, and the results of the trocar passing property are shown in Table 2.

### (Example 5)

A saccharide-added γ-PGA sheet (γ-PGA 33.3% by weight, Glu 66.7% by weight) which is the sheet-like hemostatic material according to Example 5 was produced in the same manner as in Example 4 except that a γ-PGA-saccharide solution (1% γ-PGA 2% Glu solution) of 1% by weight γ-PGA and 2% by weight glucose by adding glucose so as to be 2% by weight based on 1% by weight γ-PGA solution was prepared.

With respect to the saccharide-added γ--PGA sheet, the sheet state and the trocar passing property were respectively evaluated by the above-mentioned methods. The results of the sheet state are shown in Table 1, and the results of the trocar passing property are shown in Table 2.

### (Example 6)

A saccharide-added γ-PGA sheet (γ-PGA 50% by weight, Suc 50% by weight) which is the sheet-like hemostatic material according to Example 6 was produced in the same manner as in Example 4 except that a γ-PGA-saccharide solution (1% γ-PGA 1% Suc solution) of 1% by weight γ-PGA and 1% by weight sucrose was prepared by adding sucrose (Suc) so as to be 1% by weight based on 1% by weight γ-PGA solution.

With respect to the saccharide-added γ-PGA sheet, the sheet state and the trocar passing property were respectively evaluated by the above-mentioned methods. The results of the sheet state are shown in Table 1, and the results of the trocar passing property are shown in Table 2.

### (Example 7)

A saccharide-added γ-PGA sheet (γ-PGA 50% by weight, HA 50% by weight) which is the sheet-like hemostatic material according to Example 7 was produced in the same manner as in Example 4 except that a γ-PGA-saccharide solution (1% γ-PGA 1% HA solution) of 1% by weight γ-PGA and 1% by weight hyaluronic acid was prepared by adding 1% by weight hyaluronic acid (HA) to 1% by weight γ-PGA solution.

With respect to the saccharide-added γ-PGA sheet, the sheet state and the trocar passing property were respectively evaluated by the above-mentioned methods. The results of the sheet state are shown in Table 1, and the results of the trocar passing property are shown in Table 2.

### (Comparative Example 4)

A γ-PGA sheet (y-PGA: 100% by weight) which is the sheet-like hemostatic material according to Comparative Example 4 was produced in the same manner as in Example 4 except that a 1% γ-PGA solution was used in place of the γ-PGA-saccharide solution. With respect to this γ-PGA sheet, the sheet state and the trocar passing property were respectively evaluated by the above-described methods. The results of the sheet state are shown in Table 1, and the results of the trocar passing property are shown in Table 2.

### (Comparative Example 5)

Using TachoSil (registered trademark, CSL Behring Co., Ltd.) of 3.0 cm × 2.5 cm as the sheet-like hemostatic material according to Comparative Example 5, the trocar passing property was evaluated as described above. The results are shown in Table 2.

**[Table 3]**

| | | Examples | | | | Comparative Example 4 |
|---|---|---|---|---|---|---|
| | | **4** | **5** | **6** | **7** | |
| γ-PGA [%] | | **50** | **33.3** | **50** | **50** | **100** |
| Saccharide [%] | | **50** | **66.7** | **50** | **50** | **0** |
| | | **Glu** | **Glu** | **Suc** | **HA** | |
| State | Warpage | No | No | No | No | Yes |
| | Crack at bending | No | No | No | No | Yes |

**[Table 4]**

| Trocar passing property | | | | | |
|---|---|---|---|---|---|
| Example 4 | Example 5 | Example 6 | Example 7 | Comparative Example 4 | Comparative Example 5 |
| γ-PGA 50% | γ-PGA 33.3% | γ-PGA 50% | γ-PGA 50% | γ-PGA100% | TachoSil® |
| Glu 50% | Glu 66.7% | Suc 50% | HA 50% | | |
| Good | Good | Good | Good | Poor | Poor |
| The sheet in a roll form easily passes through the trocar. | The sheet in a roll form easily passes through the trocar. | The sheet in a roll form easily passes through the trocar. | The sheet in a roll form easily passes through the trocar. | The sheet cracks and cannot be rolled into a roll shape. | If pushed in a crushed roll shape, the sheet passes through the trocar but breaks. |

### (Example 8)

An aqueous γ-PGA-HA solution containing 60% by weight of γ-PGA and 40% by weight of HA was prepared, filled in a rectangular metal container in a predetermined amount, and frozen at -20°C for about 12 hours. The obtained frozen product was freeze-dried in a freeze dryer (product name: FDU-2100, manufactured by Tokyo Rikakikai Co., Ltd.,) under reduced pressure (about 13 Pa (1 Torr) or less) for about 24 hours. As a result, a saccharide-added γ-PGA sheet formed as a sponge-like sheet was prepared. A crosslinked collagen (C-Col) sheet was laminated as a covering layer on one surface of the saccharide-added γ-PGA sheet and then pressure-bonded. The weight ratio of the saccharide-added γ-PGA sheet to the crosslinked collagen sheet was set to 70 : 30, thereby to produce a sheet-like hemostatic material having a two-layer structure according to Example 8.

Using this sheet-like hemostatic material, its adhesiveness property and hemostatic performance were confirmed by an animal experiment. A swine was used as an experimental animal and the chest of the swine was incised under continuous anesthesia to expose the heart. Then, a part of the myocardial portion was incised to cause bleeding, and the sheet-like hemostatic material according to Example 8 was affixed to the bleeding site, and thereafter the situation of bleeding was checked.

### (Examples 4 to 8 and Comparative Examples 4 to 5)

As is apparent from Table 1, the sheet-like hemostatic materials according to Examples 4 to 7 had no warpage and no cracks at the time of bending and thus had good flexibility. In contrast, in the sheet-like hemostatic material according to Comparative Example 4, warpage as well as cracking occurred. Therefore, any saccharide of monosaccharides (glucose), oligosaccharides (sucrose), and polysaccharides (hyaluronic acid) can be added to γ-PGA to form a sheet (sheet), thereby imparting flexibility to the sheet. In addition, as a result of Example 8, it was confirmed that a saccharide-added γ-PGA sheet according to the present disclosure can realize good adhesiveness and good hemostatic performance merely by affixing to the biological tissue and lightly pressing it.

Further, as is clear from Table 2, it is understood that the sheet-like hemostatic materials according to Examples 4 to 7 can easily pass through the trocar by making them into, for example, a roll shape. On the other hand, the γ-PGA sheet (Comparative Example 4) to which no saccharide was added was not flexible and cracked when the sheet was bent, resulting in failure to form a roll. In addition, in the TachoSil (Comparative Example 5), it was barely possible to pass through the trocar by making the TachoSil into a crushed roll shape, but the TachoSil was damaged.

As described above, the sheet-like hemostatic material according to the present disclosure is more excellent in hemostatic performance and adhesive performance without containing fibrinogen than the conventional one and can realize good flexibility and good handleability.

From the above description, many modifications and other embodiments of the present invention will be apparent to those skilled in the art. Accordingly, the above description is to be construed as illustrative only and is provided for the purpose of teaching those skilled in the art the best mode of carrying out the present invention. It is possible to substantially change the details of structure and/or function of the present invention without departing from the spirit.

### Industrial Applicability

The present invention can be widely and preferably used in the field of a sheet-like hemostatic material and a method of producing the same.

### Reference Signs List

- 10A to 10E: Sheet-like hemostatic materials
- 11: Adhesive layer
- 12: Covering layer
- 13: Intermediate layer
- 111: Partial adhesive layer (sponge shape)
- 121: Partial covering layer
- 121a to 121c: Partial covering layers
- 141: Partial adhesive layer (nonporous layer)

## Claims

1. A sheet-like hemostatic material provided with an adhesive layer which serves as an affixing surface to a bleeding site and which is formed from at least poly-γ-glutamic acid, **characterized by** a covering layer which is provided on an opposite surface of the affixing surface and is formed from at least one biodegradable material other than the poly-γ-glutamic acid, wherein at least one of the adhesive layer and the covering layer is a single sheet.

2. The sheet-like hemostatic material according to claim 1, wherein the covering layer is formed from at least a collagen.

3. The sheet-like hemostatic material according to claim 2, wherein the collagen is a crosslinked collagen.

4. The sheet-like hemostatic material according to any of claims 1 to 3, wherein the adhesive layer is a sponge-like sheet when the adhesive layer is the single sheet.

5. The sheet-like hemostatic material according to any of claims 1 to 4, wherein when the adhesive layer is a partial layer which is not the single sheet, the adhesive layer is a sponge-like sheet formed from at least poly-γ-glutamic acid or a nonporous layer.

6. The sheet-like hemostatic material according to any one of claims 1 to 5, wherein the covering layer is a sponge-like sheet formed from at least a crosslinked collagen.

7. The sheet-like hemostatic material according to any of claims 1 to 6, wherein both the adhesive layer and the covering layer are each a single sheet, or the covering layer is a partial layer provided partially on the opposite surface.

8. The sheet-like hemostatic material according to any one of claims 1 to 7, wherein at least a part of the adhesive layer and the covering layer has different colors from each other.

9. The sheet-like hemostatic material according to any one of claims 1 to 8, wherein the adhesive layer is in a range of 10 to 90% by weight based on a total weight of the sheet-like hemostatic material.

10. The sheet-like hemostatic material according to any one of claims 1 to 9, further comprising at least one intermediate layer interposed between the adhesive layer and the covering layer, wherein the respective layers are integrally fixed to each other.

11. A method of manufacturing a sheet-like hemostatic material, comprising:
a step of laminating a first layered body formed from at least poly-γ-glutamic acid and a second layered body formed from at least one biodegradable material other than the poly-γ-glutamic acid to form a laminate, and
a step of adhering or pressure-bonding the laminate to form a sheet-like hemostatic material including an adhesive layer formed from at least the poly-γ-glutamic acid and a covering layer formed from the biodegradable material, wherein at least one of the adhesive layer and the covering layer is a single sheet.

12. The method of manufacturing a sheet-like hemostatic material according to claim 11, wherein:
the first layered body is a first sponge-like porous body formed from at least poly-γ-glutamic acid, a first sponge-like sheet obtained by applying pressure to the first sponge-like porous body, or a first partial sheet which is formed from at least poly-γ-glutamic acid and is not a single sheet;
the first partial sheet is sponge-like or non-sponge-like nonporous; and
the second layered body is a second sponge-like porous body formed from the biodegradable material, a second sponge-like sheet obtained by applying pressure to the second sponge-like porous body, or a second partial sheet which is formed from the biodegradable material and is not a single sheet.

13. The method of manufacturing a sheet-like hemostatic material according to claim 12, wherein the laminate is formed by first applying pressure to the second sponge-like porous body to form the second sponge-like sheet, laminating and pressure-bonding the second sponge-like sheet to the first sponge-like porous body.
